# EUROPEAN PATENT SPECIFICATION

(11) **EP 1 588 181 B1**
(45) Date of publication and mention of the grant of the patent: **14.03.2007**
(21) Application number: 03797118.1
(22) Date of filing: 23.07.2003
(51) Int. Cl.: G01R 33/465

(54) **METHOD OF DIAGNOSING COLORECTAL ADENOMAS AND CANCER USING PROTON MAGNETIC RESONANCE SPECTROSCOPY**
VERFAHREN ZUR DIAGNOSE VON KOLOREKTALADENOMEN UND -KREBS MIT PROTONENMAGNETRESONANZSPEKTROSKOPIE
PROCEDE DE DIAGNOSTIC D'ADENOME ET DE CANCER COLORECTAL PAR SPECTROSCOPIE A RESONANCE MAGNETIQUE PROTONIQUE

(30) Priority: 19.09.2002 US 411783 P
(43) Date of publication of application: 26.10.2005
(73) Proprietor: NATIONAL RESEARCH COUNCIL OF CANADA, Ottawa Ontario K1A 0R6 (CA)
(72) Inventor: BEZABEH, Tedros, Winnipeg, Manitoba R3C 3H4 (CA)
(74) Representative: McKechnie, Neil Henry
(86) International application number: PCT/CA2003/001101
(87) International publication number: WO 2004/027419

(56) References cited:
- EP-A- 0 460 910
- WO-A-02/12879
- A.S.K. DZIK-JURASZ ET AL.: "Human rectal adenocarcinoma: demonstration of /sup 1/H-MR spectra in vivo at 1.5 T" MAGNETIC RESONANCE IN MEDICINE, vol. 47, April 2002 (2002-04), pages 809-811, XP002262149 ISSN: 0740-3194

## Description

This invention relates to a method of detecting colorectal adenomas and cancer, and in particular to a method of detecting such adenomas and cancer using proton magnetic resonance spectroscopy.

Colorectal cancer is one of the most common cancers in the U.S.A. and Canada accounting for approximately 146,000 new cases in 1999. The lifetime risk that an individual in North America will develop colorectal cancer is about 5 - 6 %. Symptoms associated with colorectal cancer, including blood in the stool, anemia, abdominal pain and alteration of bowel habits often become apparent only when the disease has advanced significantly. It is well known that prognosis for a patient depends largely on the stage of the disease at the time of diagnosis. In fact, whereas the five-year survival for a patient whose colorectal cancer is detected at an early stage is 92%, survival decreases to about 60% in patients with regional spread, and to about 6% in those with distant metastases. Accordingly, it is important to detect the precursor adenomas and cancer as early as possible to increase the chances of successful therapeutic intervention.

Screening for a disease requires that the disease be prevalent in a large segment of the population and that early detection of the disease decreases mortality and improves quality of life. Colorectal cancer meets these requirements (Mandel JS, Church TR, Ederer F, Bond JH, Colorectal cancer mortality: effectiveness of biennial screening for fecal occult blood. J Natl Cancer Inst 1999; 91:434-437 and Mandel JS, Bond JH, Church TR, Snover DC, Bradley GM, Schuman LH, Ederer F, Reducing mortality from colorectal cancer by screening for fecal occult blood. Minnesota colon cancer control study, N Eng J Med 1993;328;1365-1371) and, thus, is an ideal candidate for such a program. The natural history of colorectal cancer, namely the progression from adenoma to adenocarcinoma occurring over a number of years (5 - 15), also makes it a suitable target. The cost benefit analysis for the early detection of colorectal cancer has also been shown to be favourable (Bolin, TD. Cost benefit of early diagnosis of colorectal cancer. Scand J Gastroenterol 1996; 31 Suppl 220:142-146).

The screening technique itself also has to meet a series of criteria, such as, high sensitivity and specificity, low cost, safety and simplicity. Currently, digital rectal examination (DRE), fecal occult blood test (FOBT), barium enema and direct colon visualization (sigmoidoscopy and colonoscopy) are used for this purpose.

DRE involves examining the rectum using a finger. This method detects cancers that can be palpated and are within reach of the finger. A negative DRE provides little reassurance that a patient is free of cancer, because fewer than 10% of colorectal cancers can be palpated by the examining finger.

FOBT detects hidden blood in the stool by chemical means. Although the least expensive and the simplest, the FOBT method has low sensitivity, moderate specificity and is usually not good for early detection. According to available data, a major drawback of this technique is that more than half of the cancers discovered by this method followed by x-ray or endoscopy are usually beyond the limit of early staging. A false positive rate of 10-12% is expected when the patients tested are on an unrestricted diet. Estimates of the positive predictive value range from 2.2 to 50%. The guaiac tests have a very low sensitivity, generally around 50% (Ransohoff DF, Lang CA., Screening for colorectal cancer with the fecal occult blood test; a background paper. Ann Intern Med 1997; 126:811-822). The use of FOBT is based on the assumption that colorectal cancers are associated with bleeding. However, some colorectal cancers bleed intermittently and others not at all.

A barium enema involves an x-ray of the bowel using a contrast agent. The enema can be a single or double contrast. The main radiologic signs of malignancy include muscosal disruption, abrupt cut-off and shouldering and localized lesions with sharp demarcations from uninvolved areas. The estimated sensitivity of double contrast barium enema for cancer and large polyps is only about 65-75% and even lower for small adenomas. Despite its better diagnostic yield, double contrast barium enema has a false-negative rate of 2-18%. Moreover, the method involves exposure to radiation, the repeated use of which may not be safe. Perforation from barium enema is extremely uncommon, but when it happens it is can be fatal or leads to serious long term problems as a result of barium spillage into the abdominal cavity.

A variety of instruments (collectively called endoscopes) are used for examining the bowel. Endoscopes can be rigid or flexible with varying lengths. Flexible sigmoidoscopes are 60 cm long. A colonoscope is a 130 - 160 cm flexible viewing instrument for examining the entire colon. Biopsies are taken from suspicious looking areas while viewing the colon through the endoscope. The flexible sigmoidoscopy examination is limited to the left side of the colon and rectum. At least 1/3 of neoplastic tumors occur in areas proximal to the splenic flexure that are inaccessible by sigmoidoscopy. Colonoscopy has a high sensitivity, and remains the gold standard for visualization of the colon and the detection of neoplastic abnormalities. However, it is invasive, quite expensive, and exposes the subject to risks of bowel perforation.

Magnetic resonance spectroscopy (MRS) is a technique that has the potential to detect small and early biochemical changes associated with disease processes, and has been proven to be useful in the study of tissue biopsies from cancer patients (Smith I.C.P, Bezabeh T, Tissue NMR Ex Vivo.In: Young IR, ed Methods in Biomedical magnetic resonance imaging and spectroscopy, Chicester, UK; Wiley, 2000:891-7). It is particularly useful to detect small, mobile chemical species in a given biological sample that are of diagnostic interest. Obtaining tissue biopsies for such an examination, however, usually involves an invasive procedure.

There are a number of currently available methods for detecting cancer in its stages. Biophysical methods such as conventional X-rays, nuclear medicine, rectilinear scanners, ultrasound, CAT and MRI all play an important role in early detection and treatment of cancer. Clinical laboratory testing for tumor markers can also be used as an aid in early cancer detection. Tumor marker tests measure either tumor-associated antigens or other substances present in cancer patients which aid in diagnosis, staging, disease progression, monitoring response to therapy and detection of recurrent disease. Unfortunately, most tumor marker tests do not possess sufficient specificity to be used as screening tools in a cost-effective manner. Even highly specific tests suffer from poor predictive value, because the prevalence of a particular cancer is relatively low in the general population. The majority of available tumor marker tests are not useful in diagnosing cancer in symptomatic patients because elevated levels of markers are also seen in a variety of benign diseases. The main clinical value of tumor markers is in tumor staging, monitoring therapeutic responses, predicting patient outcomes and detecting recurrence of cancer.

United States Patents Nos. 4,912,050 and 4,918,021, issued to E.T. Fossel on March 27, 1990 and April 17, 1990, respectively disclose a technique for detecting cancer by proton nuclear magnetic resonance (NMR) of blood, blood serum or blood plasma. United States Patent No. 5,261,405, issued to the same inventor on November 16, 1993 describes an apparatus and method for automating the process.

United States Patent No. 5,318,031, issued to Mountford et al on June 7, 1997 describes a method for determining chemical states of living animal or human tissue using one-dimensional NMR and 2D-COSY (two-dimensional correlation) NMR spectroscopy, and comparing measured values to reference measurements of normal, abnormal and transitions state tissue.

C. L. Lean et al (Magn. Reson Med 20:306-311,1991; Biochemistry 3:11095-11105, 1992 and Magn Reson Med 30:525-533, 1992) describe the use of magnetic resonance spectroscopy to examine colon cells and tissue specimens.

However, a need still exists for an inexpensive, non-invasive method of detecting colorectal cancer and colorectal adenomas. The object of the present invention is to provide a relatively simple, non-invasive method of detecting colorectal adenomas and cancer which meets the above defined criteria of high sensitivity and specificity, low cost and safety.

PCT International Publication No. WO 02/12879 A2 on February 14, 202 describes a method of detecting the presence of colorectal adenomas and colorectal cancer in a patient comprising the steps of subjecting a stool sample from the patient to magnetic resonance spectroscopy; and comparing the resulting spectrum with the magnetic resonance spectra of stool from non-cancerous subjects, with observed differences in spectra being indicative of cancer or clinically significant adenomas.

The performing of spectral analysis on human stool offers a significant advantage over other methods, because the collection of the specimen is non-invasive and presents no risk to the patient. Moreover, no special processing of the sample is required prior to analysis.

An alternative to the method described in the above-referenced PCT publication in which a liquid suspension of the stool sample is subjected to magnetic resonance spectroscopy has been developed by the present inventor. The rationale behind the new method is that the diagnostic markers in a sample are more than likely small water-soluble species.

Specifically, the present invention provides to a method of detecting the presence of colorectal adenomas and colorectal cancer in a patient in which a stool sample of the patient is subject to magnetic resonance spectroscopy, and the resulting spectrum is compared with the magnetic resonance spectra of non-cancerous subjects, observed differences in spectra being indicative of cancer or clinically significant adenomas, characterized in that the stool sample from the patient is mixed with a buffer to form a liquid suspension, and the suspension is centrifuged to yield a supernatant sample, which is subjected to magnetic resonance spectroscopy.

### METHOD

A centrifuge was used to prepare supernatant samples from patients' stool. The stool was homogenized and then spun down. As mentioned above, the rationale is that the diagnostic markers in the sample are more than likely small, water-soluble species. The supernatant is believed to contain the diagnostic information and spectra are improved by removal of the solid matter.

The stool samples used were those described in the above-referenced PCT publication WO 02/12879 A2, i.e. stool samples from subjects scheduled for colonoscopy or surgery. So-called "normal" samples include those from subjects with colonic conditions that are non-neoplastic. Examples include diverticulosis, hyperplastic polyps and internal hemorrhoids.

### SAMPLE PREPARATION

Specifically, homogenated raw stool sample was removed from a -70°C freezer and placed in a biohood (at room temperature) for approximately 10-15 minutes. The sample did not thaw completely in this time, but gets slightly softer and a small piece could be chipped off easily. About 2 grams of specimen was taken using a spatula and placed in a small glass vial. After a complete thaw, 6 ml of PBS/D₂O buffer was added to the vial, making the ratio of buffer to stool approximately 3:1. The suspension was then mixed (vortexed) for 2 minutes at a speed of 5 (using Vortex-2 Genie). The suspension was poured into multiple 1.5 ml centrifuge tubes and centrifuged for 3 minutes on a bench top centrifuge unit (Biofuge Pico) at 3200 RPM. The supernatant was then separated carefully and collected in 3-4 cryovials and frozen in a -70°C freezer.

After a freezing period of at least 12 hours, vials were taken out and placed in a biohood. After a sample was completed thawed, 600 µl of it was taken and put in a 5 mm NMR tube (which already contains 50 µl of TSP/D₂O). The TSP was used as a chemical shift reference (0 ppm) and for alignment of spectra during data preprocessing.

### MRS EXPERIMENTS

All samples were run on an Avance 360 Spectrometer (Bruker Instruments) with no spinning. A standard was run every morning before the actual samples to verify that the spectrometer was working properly. Once this was done, the sample was then inserted in the magnet and the time noted. The temperature was set to 298°K and lock performed on the deuterium signal (lock power = -20dB, phase = 113.5, gain = 100). Manual shimming was performed concentrating on Z, Z², Z³, X and Y. The probe head was then tuned and matched. A new experiment was written for each sample and then the acquisition parameters were set up. Water suppression was performed by the use of the presaturation technique. The line width and O1 (water frequency) were noted for each experiment.

The acquisition parameters include: pulse program Zgpr, temperature, TE = 298°K; transmitter frequency, SF01 = 360.3316979 MHz; number of scans, NS = 32; dummy scans, DS = 2; 90 degree pulse width, P1 = 9 µsec; presaturation power level, PL9 = 40dB; time domain data points, TD = 16K; size of real spectrum, Sl = 16K; interscan delay, D1 = 3.0 sec; spectral width, SW = 4990.02 Hz; acquisition time, AQ = 1.64; delay time, D9 = 60 ms.

### DATA PROCESSING

A total of 271 samples (MR spectra) have been fully analyzed to date using the three stage statistical classification strategy. Two-hundred and forty-five of these (147 normal, 98 cancer and/or adenomas) were used (in the training set) to develop the classifier. The overall accuracy on the training data (including fuzzy classifications) was 87%. The overall accuracy on the validation set (n = 26; 11 normal and 15 cancer/polyps) was 88.5%. The sensitivity was 93.3% and the specificity was 81.8%.

In the three stage statistical classification strategy mentioned above, the first stage is a preprocessing step, found to be essential for reliable classification. It consists of selecting from the spectra a few maximally discriminatory subregions, using an optimal region selection (ORS) algorithm, based on a genetic algorithm (GA)-driven optimization method (Nikulin A E, Dolenko B, Bezabeh T, Somorjai R L, NMR in Biomedicine 11, 209-217 (1998), "Near-optimal region selection for feature space reduction: Novel preprocessing methods for classifying MR spectra" Bezabeh, T et al. The use of ¹H Magnetic Resonance Spectroscopy in Inflammatory Bowel Disease: Distinguishing Ulcerative Colitis From Crohn's Disease, Am. J. Gastroenterol 2001; 96: 442-448 and Somorjai, R.L. et al, Distinguishing Normal from Rejecting Renal Allographs: Application of a Three-Stage Classification Strategy to MR and IR Spectra of Urine, Vibrational Spectroscopy 28 (1) 97-102 (2002)). For reliability of classification, the number of these subregions should be an order of magnitude smaller than the number of samples to be classified. To avoid the overly optimistic classification results that a straight resubstitution approach would give, the inventors named in the above-referenced PCT publication WO 02/12879 A2 developed a cross-validation method, using a bootstrap methodology.

The bootstrap method repeatedly partitions (with replacement) the data into approximately equal sized random training and test subsets. For each of the random training subsets an optimal classifier is found, and its accuracy validated on the random test subset. The process is repeated a number of times (1000 times at the less critical ORS preprocessing stage, 5000 times for the final classifier). Once the optimal subregions are identified, the second stage finds the ultimate classifier as the weighted average of the classifier coefficients of the 5000 individual component classifiers. This approach effectively uses all n samples. A standard multivariate statistical method, Linear Discriminant Analysis (LDA) is the choice for all classifiers, at all stages, because of its speed and robustness. The concept of crispness of a classifier is also used because the classifiers produce class probabilities. A 2-class classification of a sample is called crisp if the class assignment probability for that sample is >75%.

For difficult classification problems, a third stage consists of combining the outcomes of several classifiers via aggregation methods (computerized consensus diagnosis, CCD) into an overall classifier that is more reliable and accurate than the individual classifiers. The particular classifier aggregation used is Wolpert's Stacked Generalizer (WSG). WSG uses the output class probabilities obtained by the individual classifiers as input features to the ultimate classifier. For 2-class problems the number of features is 1 per classifier (with K independent classifiers this gives K probabilities as input features). The overall classification quality is generally higher. The crispness of the classifier is invariably greater. This,is important in a clinical environment because fewer patients will have to be re-examined.

The multivariate analysis of the one-dimensional spectra has been extremely successful for cancer biopsies (Somorjai, R. et al, J. Mag. Reson. Imaging 6, 437 (1996)). These spectra require only minutes for acquisition, provide a wide variety of data points, and can be analyzed automatically once the diagnostic algorithm has been authenticated.

## Claims

1. A method of detecting the presence of colorectal adenomas and colorectal cancer in a patient in which a stool sample of the patient is subject to magnetic resonance spectroscopy, and the resulting spectrum is compared with the magnetic resonance spectra of non-cancerous subjects, observed differences in spectra being indicative of cancer or clinically significant adenomas, **characterized in that** the stool sample from the patient is mixed with a buffer to form a liquid suspension, and the suspension is centrifuged to yield a supernatant sample, which is subjected to magnetic resonance spectroscopy.

2. A method according to claim 1, wherein the supernatant sample is subjected to one-dimensional proton magnetic resonance spectroscopy.

3. A method according to claim 2, wherein the resulting spectrum is subjected to multivariate analysis when comprising the spectrum to the spectra of stool from non-cancerous subjects.

4. A method according to claim 3, wherein the stool sample is subjected to 360 MHz magnetic resonance spectroscopy.

5. A method according to claim 4, including the steps of selecting from the spectra resulting from the one-dimensional magnetic resonance spectroscopy maximally discriminatory subregions; repeatedly partitioning the data into approximately equal sized random training and test subsets, finding an optimal classifier for each random training subset and validating the accuracy of the optimal classifier on the random test subset; and determining the ultimate classifier as the weighted average of the classifier coefficients of a large number of individual component classifiers.

## Patentansprüche

1. Methode für das Feststellen des Vorliegens von kolorektalen Adenomen und kolorektalem Krebs bei einem Patienten, wobei eine Stuhlprobe des Patienten einer Magnetresonanzspektroskopie unterworfen und das dabei erhaltene Spektrum mit den Magnetresonanzspektren nicht an Krebs leidender Patienten verglichen wird, wobei beobachtete Unterschiede zwischen den Spektren auf Krebs oder klinisch signifikante Adenome schließen lassen, **dadurch gekennzeichnet, dass** die Stuhlpropbe des Patienten mit einem Puffermittel unter Bildung einer flüssigen Suspension vermisch und die Suspension zentrifugiert wird, um eine überstehende Probe zu ergeben, die einer Magnetresonanzspektroskopie unterworfen wird.

2. Methode nach Anspruch 1, wobei die überstehende Probe eindimensionaler Protonenmagnetresonanzspektroskopie unterworfen wird.

3. Methode nach Anspruch 2, wobei das dabei erhaltene Spektrum einer Multivariatanalyse unterzogen wird, wenn das Spektrum mit den Spektren des Stuhls nicht an Krebs leidender Patienten verglichen wird.

4. Methode nach Anspruch 3, wobei die Stuhlprobe einer Magnetresonanzspektroskopie von 360 MHz unterworfen wird.

5. Methode nach Anspruch 4, umfassend die Schritte des Auswählens aus den Spektren, die durch die eindimensionale Magnetresonanzspektroskopie erhalten werden, von maximal diskriminierenden Unterregionen; wiederholten Teilens der Daten in willkürliche Schulungs- und Test-Untergruppen ungefähr gleicher Größe, Auffindens eines optimalen Klassifikators für jede willkürliche Schulungsuntergruppe und Validierens der Genauigkeit des optimalen Klassifizierers an der willkürlichen Test-Untergruppe; und Bestimmens des endgültigen Klassifizierers als gewichteter Durchschnitt der Klassifiziererkoeffizienten einer großen Anzahl einzelner Komponentenklassifizierer.

## Revendications

1. Procédé de détection de la présence d'adénomes collo-rectaux et de cancer collo-rectal chez un patient, dans lequel un échantillon de selles du patient est soumis à la spectroscopie par résonance magnétique et le spectre résultant est comparé au spectre par résonance magnétique de sujets non cancéreux, les différences observées entre les deux spectres étant indicatrices de cancer ou d'adénomes cliniquement significatifs, **caractérisé en ce que** l'échantillon de selles du patient est centrifugé pour donner un échantillon surnageant soumis à la spectroscopie par résonance magnétique.

2. Procédé selon la revendication 1, dans lequel l'échantillon surnageant est soumis à la spectroscopie par résonance magnétique unidimensionnelle du proton.

3. Procédé selon la revendication 2, dans lequel le spectre résultant est soumis à une analyse à variables multiples par comparaison dudit spectre aux spectres de selles de sujets non cancéreux.

4. Procédé selon la revendication 3, dans lequel l'échantillon de selles est soumis à la spectroscopie par résonance magnétique à 360 MHz.

5. Procédé selon la revendication 4, comprenant les étapes suivantes : sélection de sous-régions à discrimination maximale dans le spectre résultant de la spectroscopie par résonance magnétique unidimensionnelle, partition répétée des données en sous-ensembles aléatoires de formation et d'essai de taille approximativement identique, détermination d'un classificateur optimal pour chaque sous-ensemble aléatoire de formation et validation de la précision du classificateur optimal par rapport au sous-ensemble aléatoire d'essai, détermination du classificateur idéal en tant que moyenne pondérée des coefficients de classification d'un grand nombre de composantes de classification individuelles.
